# EUROPEAN PATENT SPECIFICATION

(11) **EP 1 333 280 B1**
(45) Date of publication and mention of the grant of the patent: **17.08.2005**
(21) Application number: 03001532.5
(22) Date of filing: 23.01.2003
(51) Int. Cl.: G01N 33/533, G01N 33/543, C12Q 1/68

(54) **Method for detecting biopolymers**
Verfahren zur Bestimmung von Biopolymeren
Procédé de détection de biopolymères

(30) Priority: 05.02.2002 JP 2002027616
(43) Date of publication of application: 06.08.2003
(73) Proprietor: Hitachi Software Engineering Co., Ltd., Yokohama-shi, Kanagawa 230-0045 (JP)
(72) Inventor: Sato, Keiichi, Hitachi Softw. Engineering Co., Ltd, Tokyo 140-0002 (JP); Kuwabata, Susumu, Dep. of Materials Chem. Graduate, Suita-shi, Osaka 565-0871 (JP)
(74) Representative: Liesegang, Roland, Dr.-Ing.

(56) References cited:
- WO-A-00/17656
- WO-A-00/29617
- WO-A-00/46839
- WO-A-01/73150
- SUN B ET AL: "Microminiaturized immunoassays using quantum dots as fluorescent label by laser confocal scanning fluorescence detection" JOURNAL OF IMMUNOLOGICAL METHODS, ELSEVIER SCIENCE PUBLISHERS B.V.,AMSTERDAM, NL, vol. 249, no. 1-2, 1 March 2001 (2001-03-01), pages 85-89, XP004317473 ISSN: 0022-1759
- SHEN J ET AL: "Assembly of modified CdS particles/cationic polymer based on electrostatic interactions" THIN SOLID FILMS, ELSEVIER-SEQUOIA S.A. LAUSANNE, CH, vol. 284-285, 15 September 1996 (1996-09-15), pages 242-245, XP004078074 ISSN: 0040-6090
- SUKHORUKOV G B ET AL: "MULTILAYER FILMS CONTAINING IMMOBILIZED NUCLEIC ACIDS. THEIR STRUCTURE AND POSSIBILITIES IN BIOSENSOR APPLICATIONS" BIOSENSORS & BIOELECTRONICS, ELSEVIER SCIENCE PUBLISHERS, BARKING, GB, vol. 11, no. 9, 1996, pages 913-922, XP000965111 ISSN: 0956-5663

## Description

### FIELD OF THE INVENTION

The present invention relates to a method for detecting biopolymer that do not require modification of a sample.

### BACKGROUND OF THE INVENTION

In the detection of biopolymers, optical, electrical or magnetic modification is performed on biopolymers on the sample side, and detection is performed by detecting signals obtained therefrom. Examples of a method using optical modification include a method using a fluorescent reagent, typically represented by Cy™ dye commercially available from Amersham Pharmacia Biotech, and a method using radioisotopes (RI) using a reagent having radioactivity. Further, examples of a method using electrical modification include an electrochemical detection method using a reagent as an intercalating agent to bind specifically to double strand DNA or utilizing oxidation-reduction cycle using ruthenium complex or the like. Further, examples of a method in which modification is not performed include a method utilizing surface plasmon resonance. However, in these methods, problems remain concerning the reliability and cost of analysis and the like. On the other hand, There is also a report which achieved preparation of CdS nanoparticle chains by electrostatically immobilizing CdS nanoparticles along a DNA double strands as a template (J.Phys.Chem.B,Vol.103, No.42, 1999).

B. Sun et al. (2001) Journal of Immunological Methods 249 (2001), page 85-89, disclose an immunoassay wherein ZnS-coated CdSe quantum dots are linked to a detection antibody and are subsequently used to bind selectively to an antigen captured on the surface of a glass chip through a capture antibody. A detection method using semiconductor nanoparticles which electrostatically bind to sample biopolymers is not disclosed.

WO 00/46839 discloses CdS nanoparticles and polyphosphate-stabilized nanoparticles. The nanoparticles disclosed in WO 00/46839 are envisioned to be useful in chemical sensing, DNA-sequencing, high throughput screening, fluorescence polarization immunoassays, time-gated immunoassays, time-resolved immunoassays, enzyme-linked immunosorbent assays, filtration testing and targeted tagging.

WO 00/17656 discloses water-soluble semiconductor nanocrystals having an organic outer layer. There are no methods disclosed making use of such particles.

M. Gao et al. (1996), Thin Solid Films, 284-285, 242-245 discloses a CdS nanoparticle hydrosol wherein the particles have an anionic surface. The nanoparticles were assembled using an ionene as the oppositely-charged species.

Meanwhile, a semiconductor nanoparticle is a high performance material possessing luminescence properties and electrochemical properties which has been attracting rapidly growing interest in recent years. The future utilization of semiconductor nanoparticles in various fields is anticipated.

### SUMMARY OF THE INVENTION

The present invention is a novel method of use in detection of biopolymers that has focused on the physiochemical properties of semiconductor nanoparticles. An object of the present invention is to perform low-cost, high sensitivity detection with adequate reliability in which a semiconductor nanoparticle having a chemically modified surface is used as a detection reagent to eliminate the need for modification of a sample.

According to the present invention there is provided a method for detecting biopolymers wherein, by utilizing an electric charge possessed by a sample biopolymer and using a reagent having an adsorbing property dependent on the amount of the electric charge, modification of the sample biopolymer is not required. Also disclosed is a reagent for detecting biopolymers.

The present inventors focused their attention on the physiochemical properties of semiconductor nanoparticles, and by using a semiconductor nanoparticle having a chemically modified surface as a detection reagent, succeeded in solving the above problems to complete the present invention.

That is, the reagent for detecting biopolymers comprises semiconductor nanoparticles having positively or negatively charged functional groups exposed thereon.

Examples of a means for exposing a positively or negatively charged functional group on a semiconductor nanoparticle include chemically modifying the surface of the semiconductor nanoparticle with a thiol compound.

A preferred example of a thiol compound having the above described positively charged functional group is (2-mercaptoethyl)trimethylammonium. Further, a preferred example of a thiol compound having the above described negatively charged functional group is 2-mercaptoethanesulfonic acid.

A material of the above semiconductor nanoparticle is not limited, and preferred examples thereof include ZnO, ZnS, ZnSe, ZnTe, CdO, CdS, CdMnS, CdSe, CdMnSe, CdTe, CdMnTe, HgS, HgSe, HgTe, InP, InAs, GaN, GaP, GaAs, TiO₂, WO₃, PbS, and PbSe.

According to the present invention, a -SH group of a thiol compound substitutes for an S, O, Se, Te, P, As, or N atom or the like of the surface of a semiconductor nanoparticle to chemically modify the surface of the semiconductor nanoparticle.

Further, according to the method for detecting biopolymer of the present invention, the presence of binding with a probe biopolymer and the amount of binding is detected by electrostatically binding a semiconductor nanoparticle on which a positively or negatively charged functional group is exposed to a negative or positive charge of a sample biopolymer.

Detection of the presence of binding with the probe biopolymer and the amount of binding can be performed by detecting an optical signal, electrochemical signal, or a signal generated by a combination of those two types of signals.

Further, it is possible to detect the presence of a sample biopolymer bound to a probe biopolymer immobilized on a substrate and the amount of binding.

Moreover, it is possible to detect the presence of a sample biopolymer bound to an optically, electrically or magnetically modified probe biopolymer and the amount of binding.

Furthermore, according to the above-described reagent for detecting biopolymer and method for detecting biopolymer, the detection can be performed when the probe biopolymer is probe DNA and the sample biopolymer is sample DNA.

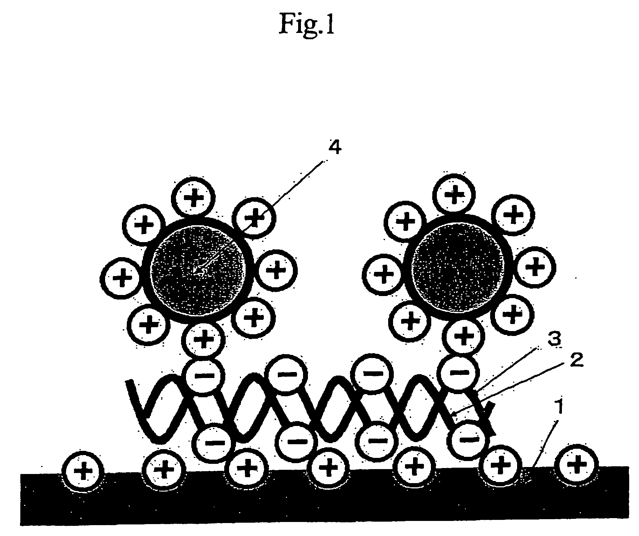

The mechanism for detecting a biopolymer according to the present invention will now be explained referring to Fig. 1. In Fig. 1, by binding between a positive charge of a surface substrate 1 forming a planar shape or bead shape and a negative charge of a phosphate side-chain of a probe DNA 2, probe DNA 2 is immobilized to the substrate 1. Probe DNA 2 and a sample DNA 3 hybridize to each other by hydrogen bonding. As a result, a negative charge of a phosphate side-chain of sample DNA 3 rises. A positively charged semiconductor nanoparticle 4 binds to the negative charge of sample DNA 3, and from the amount of the binding, information relating to hybridized sample DNA 3 is provided as a signal.

In the case of Fig. 1, probe DNA 2 is negatively charged and semiconductor nanoparticle 4 is positively charged, however a case in which the charges are the reverse thereof may also be employed. In proteins and the like, an isoelectric point exists and the charge of sample DNA 3 is changed to positive or negative in accordance with a high or low PH. When the charge of sample DNA 3 is positive, semiconductor nanoparticle 4 having a negative charge may be used.

In the present invention, a conventionally known semiconductor nanoparticle can be used. Since a semiconductor nanoparticle of a particle size of 10 nm or less is present in a transition region of a bulk semiconductor crystal and molecule, it displays physiochemical properties that are different to each thereof. In this kind of region, by expression of a quantum size effect, an energy gap increases along with a decrease in particle size. Further, accompanying this, degeneracy of an energy band observed in a bulk semiconductor breaks up, orbit is dispersed, and the lower end of a conduction band shifts to the negative side and the upper end of a valence band shifts to the positive side. In recent years, research relating to practical application utilizing this kind of characteristic is being performed at a rapid pace. In the present invention, this characteristic is utilized in practical application as a reagent for detecting biopolymers.

Examples of a material of the semiconductor nanoparticle include ZnO, ZnS, ZnSe, ZnTe, CdS, CdMnS, CdSe, CdMnSe, CdTe, CdMnTe, HgS, HgSe, HgTe, InP, InAs, GaN, GaP, GaAs, TiO₂, WO₃, PbS, and PbSe.

### BRIEF DESCRIPTION OF THE DRAWINGS

Figure 1 is a schematic illustrating detection of DNA according to the present invention.

### EXAMPLES

An example of a method of fabricating a semiconductor nanoparticle on which a positively charged functional group is exposed is hereafter described. Although a method of adjusting a CdS nanoparticle using the reverse micelle technique is described here, the method for adjusting and modifying a nanoparticle is not limited thereto, and a method of fabrication using photo-etching or the like may be similarly employed.

### Preparation of thiocholine aqueous solution

Dissolve 350 mg of Thiocholine iodaide ((2-mercaptoethyl) trimethylammonium iodide) in 1.2 cm³ of 2 mol·dm⁻³ HCl aqueous solution saturated with nitrogen, and let stand at room temperature for 12 hours. To this solution, add 0.2 cm³ of 28% ammonia water in a nitrogen atmosphere and neutralize, to produce alkalescent 0.86 mol·dm⁻³ thiocholine ((2-mercaptoethyl)trimethylammonium) aqueous solution. By modifying a nanoparticle surface with this solution, a thiocholine-modified CdS nanoparticle having a positive charge on the particle surface is prepared.

### Method of adjusting thiocholine-modified CdS nanoparticle

A method of adjusting a thiocholine-modified CdS nanoparticle having a positive charge on the surface of the particle by the inverse micelle technique is now described. The inverse micelle technique synthesizes a metal nanoparticle by reducing metal ion inside a minute space encased by a surfactant, and this technique is often used in synthesis of nanoparticles of gold, silver and the like.

14 g of sodium bis(2-ethylhexyl)sulfosuccinate (AOT) and 4 cm³ of ultrapure water are added to 200 cm³ of n-heptane, and the mixture is stirred for 40 min to prepare an AOT inverse micelle solution. The solution is divided into two parts of 120 cm³ and 80 cm³, respectively. To the former is added 0.48 cm³ of 1.0 mol·dm⁻³ Cd(ClO₄)₂ aqueous solution, and to the latter is added 0.32 cm³ of 1.0 mol·dm⁻³ NaS aqueous solution, and both solutions are then stirred until uniform. Thereafter, the two solutions are mixed, and stirring is performed for a further 1 hour to prepare a inverse micelle colloidal solution containing CdS nanoparticles. Further, by adding 0.47 cm³ of 0.86 mol·dm⁻³ thiocholine aqueous solution thereto while stirring vigorously, and further stirring overnight, the surface of the CdS nanoparticles is chemically modified with thiocholine.

### Water-solubilization of thiocholine-modified CdS nanoparticle

Add methanol to the inverse micelle solution containing CdS nanoparticles subjected to surface modification with thiocholine, and after disintegrating the inverse micelles, perform centrifugation to obtain precipitate of surface-modified CdS nanoparticles. Further, after washing several times with heptane and ethanol, respectively, add 6.0 mol·dm⁻³ tetramethylammonium chloride aqueous solution or saturated aqueous NaCl solution to conduct ion exchange of AOT present on the nanoparticle surface with chloride ion. The thus-obtained CdS nanoparticles are water-soluble.

### Refining of thiocholine-modified CdS nanoparticle

Refining of CdS nanoparticles is conducted by sedimentation by adding a nonaqueous solvent. First, ethanol is added to the obtained unrefined CdS nanoparticle aqueous solution, to reprecipitate the CdS nanoparticles. An operation of recovering only the precipitate, adding ultrapure water thereto and dissolving in water again, and then precipitating with ethanol, is repeated a further two times. Thereafter, the same operation is performed using 2-propanol and ultrapure water, to completely remove AOT. Ultrafiltration is then conducted to remove co-existing salts such as tetramethylammonium chloride and CdS of a small particle size, thus refining CdS nanoparticles that are more monodispersed.

### Modification method

An example of application to a DNA microarray is now described. The DNA microarray method is a method in which a large number of known DNA probes are chemically immobilized on a substrate and sample DNA to be assayed is then introduced on top of the probes, and the sequence characteristics of the sample are known from the existence of binding between the probe DNA and sample DNA and the amount of binding. Conventionally, a general method for determining the existence of DNA binding and the binding amount is one in which modification of a sample is performed using a fluorescent substance or radioactive substance, and the existence of binding and the binding amount is then determined by optically detecting such substance. A feature of the present invention is that, as it is not necessary to modify the sample beforehand, pretreatment of a sample by RNA reverse transcription reaction or PCR reaction is not required.

Instill sample DNA solution onto a DNA microarray and let stand with a cover glass. Then, using CHBIO (manufactured by Hitachi Software Engineering Co. Ltd.), allow reaction for 16 hours at 65°C in a sealed environment. Following reaction, extract the slide glass and immerse the slide glass in a 2x SSC 0.1% SDS solution and remove the cover glass, and then immerse for 2 hours in a 2x SSC 0.1% SDS solution containing CdS semiconductor nanoparticles at a concentration of 1.2 x 10¹⁷ mol·dm⁻³. CdS semiconductor nanoparticles may be mixed with sample DNA at the time of sample preparation. In that case, the above-described immersion is not required. Thereafter, shake in 2x SSC 0.1% SDS solution at room temperature for 20 min, and then shake in 0.2x SSC 0.1% SDS solution at room temperature for 20 min. Further, to remove nonspecific adsorption sample, shake in 0.2x SSC 0.1% SDS solution at 55°C for 20 min, and repeat the same operation once. Thereafter, conduct shaking several times in 0.2x SSC 0.1% SDS solution at room temperature, and then conduct shaking several times in respective solutions of 0.2x SSC and 0.05x SSC at room temperature. The above immersion and washing steps are all performed using a staining jar. Thereafter, analyze the slide glass using a fluorescence microscope.

### Detection method

The method of detection can be carried out according to an existing method. Specific examples include an optical detection method, an electrochemical detection method, and the like. However, since semiconductor nanoparticles possess both optical properties and electrochemical properties, a significant feature of the invention is that it is applicable to an integrated detection system such as a method that conducts excitation electrochemically and performs detection optically, or a method that conducts excitation optically and performs detection electrochemically.

In the above method, a probe may be of any form. While the present description concerns a DNA microarray, the invention can also be utilized in a method using beads, which has been attracting attention in recent years. Luminex 100 (manufactured by Luminex) is a system which involves immobilization of a probe to a bead stained with a fluorescent substance, reaction thereof with a sample modified with a fluorescent substance or the like in a solution of a microtube or the like, and contrasting of a fluorescent signal of the bead and a fluorescent signal of the sample to conduct analysis. In this case, as with a DNA microarray, the present invention can be applied to modification of a sample.

Further, the present invention is not limited to DNA and can be applied to various biopolymers. For example, in analysis of proteins, since a protein has a positive charge or negative charge according to the kind thereof, it is possible to perform modification of a protein by utilizing such properties to bind a nanoparticle to a protein sample by the method according to the present invention. Moreover, in future, when using an artificially synthesized probe, such as a peptide nucleic acid (PNA) or locked nucleic acid (LNA), detection of even higher sensitivity can be carried out.

### Effect of the Invention

By applying semiconductor nanoparticles having a positive charge or negative charge to biopolymer detection techniques, reaction and analysis are enabled that do not require modification of sample biopolymer by RNA reverse transcription reaction or PCR reaction or the like, which have been required conventionally. Further, the present invention can be applied to systems that have been generally used up to now.

## Claims

1. A method for detecting biopolymer "in vitro" that detects the presence of binding between a sample biopolymer and a probe biopolymer and the amount of binding by electrostatically binding a semiconductor nanoparticle on which a functional group having a positive or negative charge is exposed to a negative or positive charge of the sample biopolymer.

2. The method for detecting biopolymer according to claim 1, wherein the presence of binding and the amount of binding is detected by an optical signal, an electrochemical signal, or a signal generated by a combination of those two types of signals.

3. The method for detecting biopolymer according to claim 1 or 2, wherein the presence of a sample biopolymer bound to a probe biopolymer immobilized on a substrate and the amount of binding is detected.

4. The method for detecting biopolymer according to one of claims 1 to 3, wherein the presence of a sample biopolymer bound to a probe biopolymer that was modified optically, electrically or magnetically and the amount of binding is detected.

5. The method for detecting biopolymer of one of claims 1 to 4, wherein the probe biopolymer is probe DNA and the sample biopolymer is sample DNA.

6. The method for detecting biopolymer according to one of claims 1 to 5, wherein the sample biopolymer is sample protein.

## Patentansprüche

1. Verfahren zum Nachweisen von Biopolymer *"in vitro",* welches das Vorhandensein einer Bindung zwischen einem Proben-Biopolymer und einem Sonden-Biopolymer sowie den Umfang der Bindung durch elektrostatisches Binden eines Halbleiter-Nanopartikels, an welchem eine funktionelle Gruppe mit einer positiven oder negativen Ladung einer negativen oder positiven Ladung des Proben-Biopolymers ausgesetzt ist, nachweist.

2. Verfahren zum Nachweisen von Biopolymer nach Anspruch 1, wobei das Vorhandensein der Bindung und der Umfang der Bindung durch ein optisches Signal, ein elektrochemisches Signal oder ein Signal, das durch eine Kombination aus diesen beiden Typen von Signalen erzeugt wird, nachgewiesen wird.

3. Verfahren zum Nachweisen von Biopolymer gemäß Anspruch 1 oder 2, wobei das Vorhandensein eines Proben-Biopolymers, das an ein Sonden-Biopolymer, das an einem Substrat immobilisiert ist, gebunden ist, und der Umfang der Bindung nachgewiesen wird.

4. Verfahren zum Nachweisen von Biopolymer gemäß einem der Ansprüche 1 bis 3, wobei das Vorhandensein eines Proben-Biopolymers, das an ein Sonden-Polymer, das optisch, elektrisch oder magnetisch modifiziert ist, gebunden ist, und der Umfang der Bindung nachgewiesen wird.

5. Verfahren zum Nachweisen von Biopolymer nach einem der Ansprüche 1 bis 4, wobei das Sonden-Biopolymer Sonden-DNA ist und das Proben-Biopolymer Proben-DNA ist.

6. Verfahren zum Nachweisen von Biopolymer gemäß einem der Ansprüche 1 bis 5, wobei das Proben-Biopolymer Proben-Protein ist.

## Revendications

1. Procédé de détection d'un biopolymère "in vitro" qui détecte la présence d'une liaison entre un biopolymère échantillon et un biopolymère sonde et la quantité de liaison par liaison électrostatique d'une nanoparticule de semiconducteur sur laquelle un groupe fonctionnel ayant une charge positive ou négative est exposée à une charge négative ou positive du biopolymère échantillon.

2. Procédé de détection d'un biopolymère selon la revendication 1, dans lequel on détecte la présence de liaison et la quantité de liaison par un signal optique, un signal électrochimique ou un signal produit par une combinaison de ces deux types de signaux.

3. Procédé de détection d'un biopolymère selon la revendication 1 ou 2, dans lequel on détecte la présence d'un biopolymère échantillon lié à un biopolymère sonde immobilisé sur un substrat et la quantité de liaison.

4. Procédé de détection d'un biopolymère selon l'une quelconque des revendications 1 à 3, dans lequel on détecte la présence de biopolymère échantillon lié à un biopolymère sonde qui a été modifié optiquement électriquement ou magnétiquement et la quantité de liaison.

5. Procédé de détection d'un biopolymère selon l'une des revendications 1 à 4, dans lequel le biopolymère sonde est un DNA sonde et le biopolymère échantillon est un DNA échantillon.

6. Procédé de détection d'un biopolymère selon l'une des revendications 1 à 5, dans lequel le biopolymère échantillon est une protéine échantillon.
